# DEMANDE DE BREVET EUROPEEN

(11) **EP 1 352 634 A1**
(43) Date de publication de la demande: **15.10.2003**
(21) Numéro de dépôt: 03290859.2
(22) Date de dépôt: 07.04.2003
(51) Int. Cl.: A61K 7/13

(54) **Utilisation de particules métalliques organomodifiées pour le traitement de fibres kératiniques humaines**

(30) Priorité: 08.04.2002 FR 0204354
(71) Demandeur: L'OREAL, 75008 Paris (FR)
(72) Inventeur: Vic, Gabin, 60200 Compiegne (FR); Livoreil, Aude, 75006 Paris (FR); Giroud, Franck, 92110 Clichy (FR)
(74) Mandataire: Casalonga, Axel

(57) **Abrégé**

L'invention concerne l'utilisation d'une suspension de nanoparticules métalliques organomodifiées portant à leur surface une monocouche autoassemblée de composés organosoufrés pour la coloration et/ou le traitement des fibres kératiniques humaines, un procédé de coloration et/ou de traitement des fibres kératiniques humaines, comprenant l'application, sur les fibres kératiniques, d'une telle suspension, ainsi que des compositions cosmétiques servant à mettre en oeuvre un tel procédé.

## Description

La présente invention concerne l'utilisation pour la coloration et/ou le traitement des fibres kératiniques humaines, en particulier des cheveux, d'une suspension de nanoparticules métalliques organomodifiées, portant à leur surface une monocouche autoassemblée (en anglais : *self-assembled monolayer*) de groupes organosoufrés, ainsi que des compositions cosmétiques contenant une telle suspension.

La demande de brevet EP 1 064 918 décrit l'application, sur les cheveux, de nanoparticules métalliques en suspension dans une composition limpide, en vue de conférer aux cheveux traités un aspect brillant. Le dépôt des nanoparticules est décrit comme un procédé d'adsorption physico-chimique qui ne permet pas d'obtenir des dépôts rémanents, c'est-à-dire des dépôts résistant à l'élimination par les shampooings.

La demanderesse a découvert qu'il était possible d'augmenter la rémanence de nanoparticules métalliques à la surface des cheveux et de conserver ainsi leur effet cosmétique même après plusieurs shampooings, en utilisant non pas des nanoparticules métalliques "nues" telles que décrites dans EP 1 064 918 mais des nanoparticules "organomodifiées", c'est-à-dire des nanoparticules portant à leur surface des groupes organiques.

De telles nanoparticules organomodifiées ont en effet été récemment synthétisées et décrites, entre autres, dans les publications suivantes :
· Synthesis and Characterization of Carboxylate-Modified Gold-Nanoparticle Powders Dispersible in Water, Langmuir, 1999, 15, 1075 - 1082.
· Comparative Study of Dodecanethiol-Derivatized Silver Nanoparticles Prepared in One-Phase and Two-Phases Systems, Langmuir, 1998, 14, 226 - 230.

Les nanoparticules métalliques décrites dans ces publications et utilisées pour la présente invention sont caractérisées par le fait qu'elles portent à leur surface une monocouche de composés organosoufrés formant une structure communément appelée "monocouche autoassemblée" (en anglais : *self-assembled monolayer*) (voir l'article de A. Ulman, Chem. Rev., 1997, 96, 1533).

La demanderesse a constaté que les particules métalliques ainsi "habillées" d'une monocouche de composés organiques sont nettement plus faciles à manipuler et à disperser dans les différents solvant liquides en vue de la préparation d'une composition cosmétique.

La diversité des groupes organiques susceptibles d'être ainsi fixés à la surface des nanoparticules métalliques permet d'envisager plusieurs voies d'immobilisation des particules à la surface des fibres kératiniques humaines telles que
- la fixation par liaison covalente ou par adsorption des groupes organiques de la couche autoassemblée directement sur la fibre kératinique,
- la fixation par liaison covalente ou par adsorption des groupes organiques de la couche autoassemblée sur un polymère filmogène préalablement déposé à la surface de la fibre kératinique,
- le dépôt d'un mélange contenant à la fois les particules organomodifiées et un polymère filmogène, éventuellement réticulable *in situ*,
- le dépôt, sur les fibres kératiniques, de nanoparticules portant des fonctions susceptibles de réagir entre elles de manière à former autour de la fibre une couche de matériau réticulé,
ou encore,
- le dépôt, sur la fibre kératinique, de particules métalliques organomodifiées enrobées en plus par une ou plusieurs couche(s) de polymère fixée(s) par adsorption ou par liaison covalente à la couche de composés autoassemblés.

Toutes ces voies d'immobilisation mettent à profit la diversité des groupes organiques susceptibles d'être fixés à la surface des particules métalliques. Cette diversité permet de modifier avec une grande liberté les différents paramètres de surface tels que la charge électrique ou le caractère hydrophobe, de moduler ainsi les forces d'interaction avec le substrat cosmétique et d'augmenter en particulier la rémanence du dépôt de particules métalliques.

La présente invention a par conséquent pour objet l'utilisation d'une suspension de nanoparticules métalliques organomodifiées portant à leur surface une monocouche autoassemblée de composés organosoufrés, dans un milieu cosmétiquement acceptable, pour la coloration et/ou le traitement des fibres kératiniques humaines, et plus particulièrement pour améliorer la brillance des fibres kératiniques, pour faciliter la mise en forme des fibres kératiniques, pour protéger les fibres kératiniques contre le rayonnement solaire et/ou pour conditionner les fibres kératiniques.

En effet, la fixation de nanoparticules métalliques organomodifiées sur les cheveux peut apporter un certain nombre d'effets cosmétiques tels qu'une coloration et une brillance non grasse. Par ailleurs, on peut s'attendre à un effet de coiffage dû à la modification de l'état de surface des cheveux (rugosité, charge de surface). Les particules métalliques, grâce leurs propriétés réfléchissantes, seront également capables de protéger les cheveux contre les effets néfastes du rayonnement solaire. Les nanoparticules métalliques organomodifiées permettant aussi de conditionner les fibres kératiniques.

L'invention a également pour objet un procédé de coloration et/ou de traitement des fibres kératiniques humaines, comprenant l'application, sur les fibres kératiniques, d'une suspension de nanoparticules métalliques organomodifiées portant à leur surface une monocouche autoassemblée de composés organosoufrés, dans un milieu cosmétiquement acceptable.

Comme indiqué ci-dessus, il existe un grand nombre de voies d'immobilisation des nanoparticules métalliques organomodifiées sur les fibres kératiniques, autrement dit un grand nombre de procédés d'application, qui seront décrites en détail ci-après en tant que différents modes de réalisation de l'invention.

Dans un premier mode de réalisation du procédé de l'invention, les nanoparticules métalliques organomodifiées sont fixées directement sur les fibres kératiniques par liaison covalente entre des fonctions portées par les particules et des fonctions portées par la kératine.
Les résidus de composés organosoufrés fixés à la surface des nanoparticules correspondent alors à la formule

-S-R-R¹

dans laquelle
R représente un bras espaceur choisi parmi les chaînes carbonées divalentes en C₁₋₁₀₀, linéaires, ramifiées ou cycliques, saturées ou insaturées, éventuellement interrompue par des hétéroatomes tels que le soufre, l'oxygène, l'azote, le silicium ou le phosphore, et portant éventuellement un ou plusieurs substituants tels que des groupes hydroxyles, amines, thiols, carbamates, éthers, acides, esters, amides, cyano ou uréido,
R¹ représente une fonction organique capable de réagir avec les fibres kératiniques, et l'on applique la suspension de nanoparticules organomodifiées directement sur les fibres kératiniques dans des conditions permettant la fixation covalente des nanoparticules par réaction de la fonction R¹ avec la kératine, et plus particulièrement avec les fonctions hydroxyle, amine primaire et secondaire, thiol et acide carboxylique portées par les groupes latéraux des résidus d'acides aminés de celle-ci.

R tel que défini ci-dessus représente de préférence un groupe alkylène linéaire en C₁₋₅₀ et en particulier en C₄₋₂₀.

Dans un deuxième mode de réalisation du procédé de l'invention, les particules sont également fixées par liaison covalente, mais à la différence du premier, non pas directement sur la kératine mais sur un polymère filmogène déposé préalablement sur la fibre et qui joue le rôle de "primaire" pour l'accrochage des particules.
Dans ce mode de réalisation, le procédé comprend au moins deux étapes :
- une première étape consistant à déposer sur les fibres kératiniques un polymère filmogène comprenant des fonctions réactives, choisies parmi les fonctions hydroxyle, amine primaire et secondaire, thiol, acide carboxylique et anhydride carboxylique, et
- une deuxième étape consistant à appliquer sur les fibres kératiniques entourées dudit polymère filmogène, une suspension de nanoparticules métalliques organomodifiées portant à leur surface une monocouche autoassemblée de groupes organosoufrés de formule

   -S-R-R¹
dans laquelle
R a la signification et la signification préférée indiquée ci-dessus, et R¹ représente une fonction organique capable de réagir avec les fonctions réactives hydroxyle, amine primaire et secondaire, thiol, acide carboxylique et anhydride carboxylique dudit polymère filmogène,
dans des conditions permettant la fixation covalente des nanoparticules par réaction entre la fonction R¹ et les fonctions dudit polymère filmogène.

Il existe un grand nombre de fonctions organiques capables de réagir avec les fonctions à hydrogène actif de la kératine ou du polymère filmogène déposé au préalable sur les cheveux. L'homme du métier connaît ces fonctions et leur mécanisme réactionnel (substitution, addition sur une double ou triple liaison, ouverture de cycle etc.) permettant d'aboutir à une liaison covalente, et saura également choisir les conditions réactionnelles appropriées pour permettre l'établissement d'une liaison covalente (température, pH, application d'un rayonnement, présence d'un co-réactif ou d'un catalyseur chimique ou biochimique).
On peut citer à titre d'exemples les fonctions R¹ suivantes : époxyde, aziridine, vinyle, acrylonitrile, acide (méth)acrylique, (méth)acrylate d'alkyle, acide crotonique, acide cinnamique et cinnamate d'alkyle, styrène, butadiène, vinyloxy, vinylcétone, maléate d'alkyle, maléimides, vinylsulfone, acide carboxylique, chlorure d'acide carboxylique, anhydride carboxylique, carboxylate d'alkyle, acétal, hémi-acétal, aminal, hémi-aminal, cétone, α-hydroxycétone, α-halogénocétone, lactone, thiolactone, isocyanate, thiocyanate, imine, imide (succinimides, glutimides), ester de N-hydroxysuccinimide, imidate, oxazine, oxazoline, oxazinium, oxazilinium, halogénures d'alkyle, d'aryle ou d'aralkyle, halogénure d'un carbocycle ou hétérocycle insaturé (par exemple chlorotriazine, chlorpyrimidine, chloroquinoxaline, chlorobenzotriazole), halogénure de sulfonyle, siloxane, silane, hydrazine, phénylglyoxal, aldéhyde, azlactone, imidoester, thiosulfate, diazirine, pyridylthio, amine primaire et secondaire et phénylazide.

Le polymère filmogène déposé au préalable et comportant des fonctions réactives peut être choisi par exemple parmi la polyéthylèneimine, la polylysine, les alcools polyvinyliques, le poly((méth)acrylate d'hydroxyéthyle), les hydroxyalkylcelluloses, l'acide polyacrylique, les polyvinylimidazoles, les polypropylène-imines, les polyallylamines, le chitosane, les carboxyalkylcelluloses, les aminoalkylcelluloses, les polymères dérivés d'acide ou d'anhydride maléique, fumarique et/ou itaconique, les polyamidoamines.
Parmi ces polymères, on préfère en particulier les polymères cationiques tels que les polyéthylèneimines et les polylysines, en raison de leur excellente affinité pour le substrat kératinique qui a une charge globale anionique.

Dans le deuxième mode de réalisation du procédé de l'invention décrit ci-dessus, on pourra, bien entendu, également envisager que les fonctions décrites ci-dessus pour R¹ soient portées, non pas par les composés organosoufrés de la couche autoassemblée, mais par le polymère filmogène déposé au préalable et que R¹ représente alors une fonction capable de réagir avec les fonctions du polymère.

Dans un troisième et quatrième mode de réalisation du procédé de la présente invention, les particules métalliques sont fixées non pas par liaison covalente mais par adsorption physico-chimique sur le substrat kératinique. On définit l'adsorption comme un phénomène dû à des interactions dites faibles qui mettent en oeuvre des énergies de liaison inférieures à 50 kcal/mol. Les forces de liaison englobent par exemple les interactions ioniques, les forces de van der Waals, l'interaction hydrophobe et les liaisons hydrogène.

Concrètement, dans un troisième mode de réalisation du procédé de la présente invention, les groupes organosoufrés correspondent à la formule

-S-R-R²

dans laquelle
R a la même signification et signification préférée que précédemment, R² représente une fonction organique capable d'établir des interactions faibles avec les fibres kératiniques, et l'on applique la suspension de nanoparticules organomodifiées directement sur les fibres kératiniques dans des conditions permettant l'adsorption des nanoparticules par interaction faible entre la fonction R² et la surface du cheveu.

On peut, bien entendu, également envisager, par analogie avec l'immobilisation par liaison covalente (modes de réalisation 1 et 2), que l'adsorption des particules par l'intermédiaire des fonctions R² se fait, non pas directement sur la kératine des cheveux, mais sur un polymère préalablement déposé sur ces derniers. Par conséquent, un quatrième procédé de coloration et/ou de traitement des fibres kératiniques humaines comprend
- une première étape consistant à déposer sur les fibres kératiniques un polymère filmogène et
- une deuxième étape consistant à appliquer sur les fibres kératiniques entourées dudit polymère filmogène une suspension de nanoparticules métalliques organomodifiées portant à leur surface une monocouche autoassemblée de groupes organosoufrés de formule

   -S-R-R²

   où R et R² ont la même signification que précédemment, dans des conditions permettant l'établissement d'interactions faibles entre le groupe R² et ledit polymère filmogène préalablement déposé.

Un très grand nombre de polymères filmogènes et de fonctions R² susceptibles d'établir des interactions faibles avec ledit polymère filmogène peuvent être envisagées.
On peut citer à titre d'exemple de fonctions R², les groupes dérivés d'un composé choisi parmi les acides carboxyliques et leurs sels, les amines primaires, secondaires, tertiaires ou quaternaires, les phosphates, les composés soufrés oxydés tels que les sulfones, les acides sulfoniques, les sulfoxydes et les sulfates, les fullerènes, les nanotubes de carbone, les composés aromatiques carbocycliques ou hétérocycliques, les pyrènes, les stilbènes, les ferrocènes, les carbazoles, l'uréido-pyrimidone, la mélamine, l'acide cyanurique, les phtalohydrazides, l'isoguanine, le glycolurile, l'uracile, l'acylaminopyridine, la thymine, la guanine, la cytidine, l'adénine et la ptérine.

Les polymères filmogènes utilisables dans ce procédé sont les mêmes que ceux indiqués précédemment.

Ce quatrième mode de réalisation du procédé de l'invention englobe également un mode de liaison particulier connu, surtout dans le domaine biochimique, sous le nom de liaison par "affinité". Il s'agit d'une interaction réversible, plus ou moins *spécifique*, entre un ligand et un récepteur biologique. On peut citer à titre d'exemples de tels couples ligand-récepteurs les suivants : biotine/avidine, anticorps, antigène, lectines/oligosaccharide, brins complémentaires d'ADN.
Appliqué au mode de réalisation décrit ci-dessus, l'un des partenaires, c'est-à-dire soit le ligand soit le récepteur, sera porté par le polymère et l'autre correspondra à la fonction R² portée par les composés organosoufrés autoassemblés à la surface des particules métalliques.

Dans un cinquième mode de réalisation du procédé de la présente invention, les particules métalliques enrobées d'une monocouche autoassemblée de composés organiques sont déposées sur les fibres kératiniques en même temps qu'un polymère filmogène. Après évaporation du milieu de suspension, le film séché contient les nanoparticules organomodifiées incorporées dans et retenues par le film polymérique.
Dans ce mode de réalisation, la suspension de nanoparticules métalliques organomodifiées contient donc en outre au moins un polymère filmogène à l'état dissous ou dispersé dans le milieu cosmétiquement acceptable. Les polymères appropriés sont identiques à ceux indiqués ci-dessus pour le quatrième procédé. Ils présentent de préférence une charge globale cationiques qui leur confère une bonne affinité pour la kératine.

On pourra également envisager une variante particulière de ce mode de réalisation dans laquelle le polymère filmogène est réticulé *in situ* après dépôt grâce à des fonctions réactives portées par le polymère, par exemple des doubles liaisons, ou par un agent de réticulation approprié présent dans la suspension de départ ou appliqué après celle-ci. Une telle gaine de polymère réticulée renfermant les nanoparticules métalliques organomodifiées résistera particulièrement bien à l'élimination par lavage des cheveux.

Une autre possibilité pour former une gaine réticulée rémanente contenant des nanoparticules métalliques organomodifiées consiste à utiliser des nanoparticules capables d'établir entre elles des liaisons covalentes par réaction de fonctions réactives portées par les composés organosoufrés autoassemblés en une monocouche à la surface des particules.
Dans un sixième mode de réalisation du procédé de l'invention, les composés organosoufrés portent par conséquent des fonctions susceptibles de réagir entre elles de manière à former autour de la fibre, lors de l'évaporation du milieu solvant, une couche de matériau réticulé.
On peut citer à titre d'exemples de telles fonctions, les silanes réactifs tels que les halogénosilanes ou alcoxysilanes, les siloxanes, les thiols, ou encore les doubles liaisons acryliques ou vinyliques polymérisables en présence d'un amorceur approprié.
Le dépôt obtenu sera insoluble dans l'eau et résistera bien aux shampooings, ce qui garantit la bonne durabilité des effets cosmétiques apportés par ce dépôt.

Un septième mode de réalisation du procédé de l'invention consiste à appliquer, sur les cheveux, des nanoparticules métalliques organomodifiées par une monocouche autoassemblée de composés organosoufrés et qui sont en outre enrobées d'une ou de plusieurs couche(s) de polymère(s) organique(s), identiques ou différents.
Ce polymère peut être simplement adsorbé à la surface des nanoparticules organomodifiées, ou bien il peut être fixé par liaison covalente aux composés organosoufrés autoassemblés.

La fixation par liaison covalente du polymère organique peut être obtenue de manière analogue à celle décrite pour le deuxième mode de réalisation du procédé, à savoir par réaction entre des fonctions R¹ portées par les particules et des fonctions réactives portées par le polymère.
On peut également envisager de copolymériser par voie radicalaire des particules métalliques organomodifiées portant des groupes époxydes ou des doubles liaisons vinyliques ou acryliques, de préférence acryliques, avec un ou plusieurs co-monomères vinyliques ou acryliques ou de type époxyde.
Les nanoparticules organomodifiées peuvent également fonctionner comme un amorceur radicalaire de la réaction de polymérisation qui sera ainsi incorporé dans la chaîne macromoléculaire.
L'homme du métier connaît les différents procédés de polymérisation permettant de mettre en oeuvre une telle polymérisation et l'on citera simplement à titre d'exemple préféré d'un tel procédé, la polymérisation radicalaire par transfert d'atomes (ATRP) décrite par exemple dans l'article *"Controlled Synthesis of Crosslinked Ultrathin Polymer Films by Using Surface-Initiated Atom Transfer Radical Polymerization*", publié dans *Angew. Chem., Int. Ed*., 2001, 40, 12510 - 12512.
L'encapsulation décrite dans cet article est obtenue par polymérisation radicalaire amorcée par des disulfures qui forment la monocouche autoassemblée (SAM, *self-assembled monolayer*). Un tel procédé permet d'obtenir des particules ayant une structure de type noyau métallique/enveloppe organique. L'épaisseur de l'enveloppe est généralement comprise entre 2 nm et 300 nm.
On peut citer comme amorceur radicalaire disulfure préféré, capable de former des monocouches autoassemblées, le composé de formule

(BrC(CH₃)₂COOH(CH₂)₁₁S)₂

et, comme monomère préféré, le diméthacrylate d'éthylèneglycol. On utilisera de préférence des nanoparticules d'or ou d'argent.

L'adjectif "métallique" utilisé dans la présente invention pour décrire les nanoparticules organomodifiées signifie
- soit que les nanoparticules sont constituées à 100 % d'un ou de plusieurs métaux à l'état élémentaire,
- soit que les nanoparticules comportent une couche superficielle (enveloppe) constituée à 100 % d'un ou de plusieurs métaux à l'état élémentaire, et qui entoure un noyau, ou coeur, constitué d'un matériau différent. De telles nanoparticules à structure noyau/enveloppe seront décrites plus en détail ci-après.

Les métaux formant les nanoparticules métalliques organomodifiées sont choisis de préférence parmi les métaux alcalins, les métaux alcalino-terreux, les métaux de transition, les métaux des terres rares et des alliages de ces métaux.
On préfère utiliser en particulier l'aluminium, le cuivre, le cadmium, le sélénium, l'argent, l'or, l'indium, le fer, le platine, le nickel, le molybdène, le silicium, le titane, le tungstène, l'antimoine, le palladium, le zinc, l'étain et les alliages de ces métaux, et parmi ceux-ci tout particulièrement l'or, l'argent, le palladium, le platine, le cadmium, le sélénium et les alliages de ces métaux.

Les nanoparticules métalliques organomodifiées utilisées dans la présente invention peuvent être préparées notamment selon les deux procédés de synthèse suivants :
Procédé 1 : On ajoute directement un ou plusieurs composés thiolés de formule générale R¹-R-SH à un sel métallique, tel que HAuCl₄,3H₂O ou AgNO₃, en suspension ou en solution dans un milieu aqueux ou organique, puis on procède à la réduction dudit sel métallique par addition d'un réducteur tel que le borohydrure de sodium, le thiosulfate de sodium ou le citrate trisodique. Un tel procédé est décrit par exemple dans l'article intitulé "Synthesis and Characterization of Carboxylate-Modified Gold Nanoparticle Powders Dispersible in Water", Langmuir, 1999, 15, 1075 - 1082.
Procédé 2 : On incube une suspension de nanoparticules métalliques dans une solution de thiolates et/ou de thiols et/ou de disulfures et/ou de thioéthers et/ou de xanthates et/ou de thiocarbamates et/ou de thiosulfates et/ou de thiolactones pour former une monocouche par chimisorption des dérivés thiolés la surface des nanoparticules.

La monocouche de composés thiolés peut ensuite être modifiée par
- réaction d'échange avec de nouveaux dérivés thiolés pour former une nouvelle couche de composition différente
- réaction de polymérisation utilisant les fonctions chimiques présentes à l'extrémité des composés thiolés adsorbés formant la monocouche autoassemblée,
- réaction de substitution nucléophile et électrophile,
- réaction de substitution radicalaire,
- réaction d'addition sur liaison multiple carbone-carbone ou carbone-hétéroatome,
- réaction d'élimination,
- réaction d'oxydation.
Des exemples de telles modifications de la structure chimique de la monocouche autoassemblée sont décrits par exemple dans l'article de R. Murray, Acc. Chem. Res., 2000, 33, pages 27 - 36.

Comme indiqué ci-avant, les nanoparticules métalliques utilisées dans la présente invention englobent également des nanoparticules composites de type noyau/enveloppe, constituées d'une enveloppe métallique qui entoure un noyau constitué d'un matériau différent d'un métal à l'état élémentaire.
Le noyau de telles nanoparticules peut être constitué d'un matériau minéral ou organique. Lorsqu'il s'agit d'un matériau minéral, celui-ci est choisi de préférence parmi les oxydes, oxydes dihydratés, hydroxydes, carbonates, sulfures, silicates et phosphates de silicium, calcium, magnésium, zinc, aluminium, titane, zirconium ou cérium, les micas et les nacres.
Lorsqu'il s'agit d'un matériau organique, il s'agit de préférence d'un polymère organique choisi parmi les homopolymères et copolymères de styrène, les polyorganosiloxanes, les polymères fluorés, les copolymères d'éthylène et d'acétate de vinyle, les alcools polyvinyliques, le poly(oxyde d'éthylène), la polyvinylpyrrolidone, les homopolymères et copolymères à base d'acide (méth)acrylique et/ou de (méth)acrylates d'alkyle, les polyuréthannes, les polyamides, les polycarbonates, le poly(chlorure de vinyle), le poly(acétate de vinyle), le polypropylène, le polyéthylène, le polyisobutylène, le poly(1-buténylène), les éthers de cellulose, les esters organiques de cellulose, les carboxyalkylcelluloses, les sulfates de cellulose, les sulfates de dextrane et les éthers de dextranes.
Ces polymères organiques formant le noyau des nanoparticules métalliques peuvent être réticulés à l'aide d'agents réticulants appropriés choisis en fonctions du polymère. On peut indiquer à titre d'exemples de tels agents réticulants le divinylbenzène, le glutaraldéhyde, la 1,4-bis(acyloyl)pipérazine, les carbodiimides, le N-hydroxysuccinimide, la divinylsulfone, le dithiobis(succinimidyl)propionate et le N-succinimidyl-3-(2-pyridyldithio)propionate.

Les nanoparticules métalliques organomodifiées utilisées selon la présente invention pour la coloration et/ou le traitement des fibres kératiniques présentent des propriétés de coloration intrinsèques qui dépendent non seulement de la nature du métal et des composés organosoufrés formant la monocouche autoassemblée, mais également de la taille des particules.
Ces propriétés de coloration peuvent être modifiées par greffage de groupes absorbant la lumière visible ou UV, dérivés de colorants organiques connus. Ces colorants organiques non-ioniques, cationiques, anioniques ou amphotères sont choisis par exemple parmi les colorants benzéniques nitrés, les colorants benzéniques aminés, les colorants azoïques, les colorants naphto-, benzo- ou anthraquinoniques, les colorants de type diamine aromatique, les aminophénols, les colorants phénoliques et naphtoliques, les porphyrines telles que les tétraphénylporphyrines et les métalloporphyrines, les phtalocyanines, les caroténoïdes, les flavonoïdes et différentes molécules fluorescentes telles que la fluorescéine, la rhodamine et la coumarine.

La possibilité d'apporter aux fibres kératiniques humaines une couleur et/ou un effet cosmétique particulier grâce à la fixation de nanoparticules métalliques organo-modifiées selon les différents modes de réalisation du procédé de la présente invention s'avère particulièrement intéressante en combinaison avec des traitements cosmétiques connus des cheveux, tels que la déformation permanente, la coloration d'oxydation ou la décoloration des cheveux.

Le procédé de la présente invention, mis en oeuvre avant ou après les différentes étapes des procédés de traitements connus, peut alors améliorer l'efficacité desdits traitements ou apporter un effet cosmétique particulier, spécialement adapté aux modifications chimiques et/ou physiques, souhaitables ou indésirables, qu'entraînent ces traitements.

La présente invention a par conséquent également pour objet un procédé de pré-traitement ou de post-traitement des fibres kératiniques humaines, consistant à mettre en oeuvre les procédés selon la présente invention avant ou après un traitement de coloration d'oxydation, de réduction, de décoloration, de permanente, de coiffage ou de défrisage des fibres kératiniques.

La présente invention a également pour objet une composition cosmétique contenant, dans un milieu cosmétiquement acceptable,
- au moins un principe actif cosmétique, et
- des nanoparticules métalliques organomodifiées portant à leur surface une monocouche autoassemblée de composés organosoufrés, décrites ci-dessus.

Les compositions cosmétiques de la présente invention contiennent de préférence de 0,0001 % à 50 % en poids, en particulier de 0,01 % et 5 % en poids, et idéalement de 0,05 % à 2 % en poids de nanoparticules métalliques organomodifiées.
Les principes actifs cosmétiques présents dans les compositions cosmétiques de la présente invention sont choisis par exemple parmi les vitamines, les saccharides, les oligosaccharides, les polysaccharides hydrolysés ou non, modifiés ou non, les acides aminés, les oligopeptides, les peptides, les protéines hydrolysées ou non, modifiées ou non, les polyacides aminés, les enzymes, les acides et alcools gras ramifiés ou non, les cires animales, végétales ou minérales, les céramides et les pseudo-céramides, les acides organiques hydroxylés, les filtres UV, les agents anti-oxydants et les agents anti-radicaux libres, les agents chélatants, les agents antipelliculaires, les agents régulateurs de séborrhée, les agents apaisants, les agents tensioactifs cationiques, les polymères cationiques, les polymères amphotères, les silicones organomodifiées ou non, les huiles minérales, végétales ou animales, les polyisobutènes et poly(α-oléfines), les esters gras, les polymères anioniques sous forme dissoute ou dispersée, les polymères non-ioniques sous forme dissoute ou dispersés, les agents réducteurs, les colorants capillaires ou les pigments et leurs mélanges.
Le principe actif cosmétique est présent de préférence à raison de 0,001 à 50 % en poids, en particulier de 0,01 à 20 % en poids, et idéalement de 0,1 à 10 % en poids, rapporté au poids total de la composition cosmétique.

Le milieu cosmétiquement acceptable peut contenir de l'eau et/ou un ou plusieurs solvants organiques cosmétiquement acceptables tels que les alcools inférieurs comme l'éthanol, des alcanes en C₅₋₂₀, l'acétone, la méthyléthylcétone, l'acétate de méthyle, l'acétate d'éthyle, l'acétate de butyle, le diméthoxyéthane, les polyols, les éthers de polyols, le diéthoxyéthane et les silicones volatiles.

Les compositions cosmétiques de la présente invention peuvent se présenter sous n'importe quelle forme permettant une application régulière d'une quantité suffisante de nanoparticules sur les fibres kératiniques. Il peut s'agir de compositions à rincer ou non, et notamment de lotions, de sprays aérosol, de mousses, de gels, de shampooings ou d'après-shampooings.

Les compositions cosmétiques peuvent contenir, bien entendu - en plus du milieu cosmétiquement acceptable, d'au moins un principe actif cosmétique et des nanoparticules métalliques organomodifiées - un ou plusieurs adjuvants de formulation choisis par exemple parmi les agents épaississants, les agents d'ajustement et de fixation du pH, les agents conservateurs, les agents anti-mousse, les parfums et les agents tensioactifs non-cationiques. Lorsque la composition se présente sous forme d'aérosol, elle contient bien entendu en outre un ou plusieurs agents propulseurs tels que l'air, le gaz carbonique, l'azote, le diméthyléther et les hydrocarbures éventuellement halogénés.

L'invention sera illustrée à l'aide des exemples suivants.

### Exemple 1

### Synthèse de nanoparticules d'or enrobées d'une monocouche autoassemblée de composés à fonctions carboxyliques

On prépare une solution aqueuse à 5 % (196 mg dans 4 g d'eau) de HAuCl₄, 3H₂O de chez Aldrich. On prépare une solution alcoolique d'acide mercaptosuccinique par dissolution de 187 mg (1,25 mmole) d'acide mercaptosuccinique (Aldrich) dans 100 ml de méthanol. On mélange les deux solutions à température ambiante en agitant vigoureusement à l'aide d'un agitateur magnétique. On dissout ensuite 189 mg de borohydrure de sodium (Aldrich) dans 25 ml d'eau. On ajoute cette dernière solution lentement, à une vitesse de 5 ml/minute, au mélange indiqué ci-dessus. On maintient ensuite l'agitation pendant une heure à température ambiante. La suspension obtenue ainsi est centrifugée pendant 5 minutes à 9840 g. On élimine le surnageant et l'on reprend le culot dans 100 ml d'un mélange méthanol/eau 80/20. On soumet cette suspension pendant 5 minutes à des ultrasons émis par une sonde. On répète le cycle centrifugation/remise en suspension deux fois avec un mélange méthanol/eau 80/20, puis une troisième fois avec du méthanol pur. On reprend enfin le solide (culot) dans 100 ml d'éthanol et l'on évapore sous vide à l'aide d'un évaporateur rotatif à une température de 35 °C et à une pression réduite de 20 mbars. On obtient ainsi une poudre finement dispersée de couleur marron.
Les nanoparticules métalliques carboxylées sont dispersibles dans l'eau ou dans des mélanges eau/alcool et donnent une solution colloïdale de couleur marron foncé/noir.

### Analyse élémentaire quantitative par ESCA (Elemental Spectroscopy for Chemical Analysis)

On disperse 10 mg des nanoparticules métalliques carboxylées préparées ci-dessus dans 3 ml d'une solution alcoolique à 10 % et on dépose 200 µl de cette suspension sur un cristal de silicium ayant une surface de 1 cm². Après évaporation du solvant, on analyse l'échantillon dans un analyseur VG ESCALAB MkII. Des balayages sont effectués sur les pics correspondant à Au 4f^{7/2}, C1s, O1s et S2s à une énergie constante de 20 eV.
L'analyse élémentaire révèle les teneurs suivantes (en % en atomes) pour les différents éléments suivants :
Au : 2,41 % ; O : 16,84 % ; C : 79,82 % ; S : 0,77 % et Na : 0,16.
Ces résultats confirment donc bien la présence attendue d'or, de soufre et de carbone dans les nanoparticules analysées.

### Détermination de la taille des particules par microscopie électronique à transmission (MET)

On met en suspension les nanoparticules métalliques organomodifiées dans une solution alcoolique à 10 % sous agitation par ultrasons. On dépose une goutte de cette solution sur une grille de carbone. On observe les nanoparticules à 400 kV avec un microscope électronique haute résolution Jeol 4000 EX (II) avec une résolution de 0,16 nm. Plusieurs zones qui ont été observées à un agrandissement de 400 000 montrent des particules ayant une taille comprise entre 1,25 et 6,35 nm avec une taille moyenne égale à 3,75 nm.

### Exemple 2

### Fixation des nanoparticules carboxylées directement sur les fibres kératiniques

On lave 85 mg de morceaux de cheveux blancs (longueur 200 µm) avec 1 ml de shampooing dans un tube Eppendorf d'une capacité de 1,5 ml. Après centrifugation, on élimine le surnageant et l'on rince le culot de cheveux deux fois avec 1 ml d'eau distillée. On ajoute ensuite aux cheveux 1 ml d'une suspension aqueuse des nanoparticules carboxylées préparées dans l'exemple 1 (5,2 mg/ml) et on agite pendant 1 heure à température ambiante. Après centrifugation, on élimine le surnageant et on rince 3 fois avec 1 ml d'une solution de shampooing, puis de nouveau 3 fois avec de l'eau distillée. Les cheveux sont ensuite séchés pendant 12 heures à 45 °C.
Les morceaux de cheveux ainsi traités présentent une couleur noire marquée qui est rémanente aux shampooings.
Etant donné que la très faible taille des nanoparticules ne permet pas une détection directe en microscopie électronique à balayage (MEB), on traite le dépôt d'abord avec une solution commercialisée par la société Sigma sous le nom de Silver Enhancer SE-100 qui permet d'augmenter la taille des particules par dépôt supplémentaire d'argent à leur surface.
La microscopie électronique à balayage et l'analyse EDX montrent clairement la présence de nanoparticules d'or à la surface des morceaux de cheveux.

### Exemple 3

### Fixation de nanoparticules carboxylées par adsorption sur des fibres kératiniques enrobées de polyéthylèneimine

On lave 85 mg de morceaux de cheveux blancs (longueur 200 µm) avec 1 ml de shampooing dans un tube Eppendorf d'une capacité de 1,5 ml. Après centrifugation, on élimine le surnageant et l'on rince le culot de cheveux deux fois avec 1 ml d'eau distillée. Les cheveux sont ensuite incubés pendant 1 heure à température ambiante en présence de 1 ml d'une solution aqueuse contenant 5 % de polyéthylèneimine (commercialisée par la société Bayer sous la dénomination LUPAZOL®). A la fin de cette incubation, on centrifuge, on élimine le surnageant et on rince deux fois avec 1 ml d'eau distillée. On ajoute ensuite au culot de cheveux 1 ml d'une suspension aqueuse des nanoparticules carboxylées préparées dans l'exemple 1 (5,2 mg/ml) et on agite pendant 4 heures à température ambiante. Après centrifugation, on élimine le surnageant et on rince 3 fois avec 1 ml d'une solution de shampooing puis de nouveau 3 fois avec de l'eau distillée. Les cheveux sont ensuite séchés pendant 12 heures à 45 °C.
Les morceaux de cheveux ainsi obtenus présentent une couleur noire marquée qui est rémanente aux shampooings.
La microscopie électronique à balayage et l'analyse EDX après traitement avec une solution de Silver Enhancer SE-100 (Sigma) confirment la présence de nanoparticules d'or à la surface des cheveux.

### Exemple 4

### Fixation de nanoparticules carboxylées par greffage chimique sur des fibres kératiniques enrobées de polyéthylèneimine

On lave 85 mg de morceaux de cheveux blancs (longueur 200 µm) avec 1 ml de shampooing dans un tube Eppendorf d'une capacité de 1,5 ml. Après centrifugation, on élimine le surnageant et l'on rince le culot de cheveux deux fois avec 1 ml d'eau distillée. Les cheveux sont ensuite incubés pendant 1 heure à température ambiante en présence de 1 ml d'une solution aqueuse contenant 5 % de polyéthylèneimine (commercialisée par la société Bayer sous la dénomination LUPAZOL®). A la fin de cette incubation, on centrifuge, on élimine le surnageant et on rince deux fois avec 1 ml d'eau distillée. A 1 ml d'une suspension de nanoparticules d'or carboxylées (5,5 mg/m) dans un tampon MES pH 4,7, 0,1 M, on ajoute 28 mg de 1-éthyl-3(3-diméthylaminopropyl)carbodiimide (EDC) et 20 mg de N-hydroxysuccinimide (NHS). La solution est agitée pendant 1 minute à température ambiante et ajoutée ensuite au culot de cheveux rincés. On agite la suspension obtenue pendant 4 heures à température ambiante, on centrifuge, on élimine le surnageant et l'on rince trois fois avec 1 ml d'une solution de shampooing, puis trois fois avec 1 ml d'eau distillée. Les cheveux sont ensuite séchés pendant 12 heures à 45 °C.
Les morceaux de cheveux ainsi traités présentent une couleur noire marquée qui est rémanente aux shampooings.
Après traitement avec une solution de Silver Enhancer S-100 (Sigma) les cheveux sont analysés par microscopie électronique à balayage et par EDX.

## Revendications

1. Utilisation d'une suspension de nanoparticules métalliques organomodifiées portant à leur surface une monocouche autoassemblée de composés organosoufrés, dans un milieu cosmétiquement acceptable, pour la coloration et/ou le traitement des fibres kératiniques humaines.

2. Utilisation selon la revendication 1 pour améliorer la brillance des fibres kératiniques, pour faciliter la mise en forme des fibres kératiniques, pour protéger les fibres kératiniques contre le rayonnement solaire et/ou pour conditionner les fibres kératiniques.

3. Procédé de coloration et/ou de traitement des fibres kératiniques humaines, comprenant l'application, sur les fibres kératiniques, d'une suspension de nanoparticules métalliques organomodifiées portant à leur surface une monocouche autoassemblée de composés organosoufrés, dans un milieu cosmétiquement acceptable.

4. Procédé selon la revendication 3, **caractérisé par le fait que** les résidus de composés organosoufrés fixés à la surface des nanoparticules correspondent à la formule
-S-R-R¹
dans laquelle
R représente un bras espaceur choisi parmi les chaînes carbonées divalentes en C₁₋₁₀₀, linéaires, ramifiées ou cycliques, saturées ou insaturées, éventuellement interrompues par des hétéroatomes, et portant éventuellement un ou plusieurs substituants tels que des groupes hydroxyles, amines, thiols, carbamates, éthers, acides, esters, amides, cyano ou uréido, de préférence un bras espaceur choisi parmi les groupes alkylène linéaire en C₁₋₅₀ et en particulier en C₄₋₂₀, et
R¹ représente une fonction organique capable de réagir avec les fibres kératiniques,
et que l'on applique la suspension de nanoparticules organomodifiées directement sur les fibres kératiniques dans des conditions permettant la fixation covalente des nanoparticules par réaction de la fonction R¹ avec la kératine.

5. Procédé selon la revendication 3, **caractérisé par le fait qu'**il comprend
• une première étape consistant à déposer sur les fibres kératiniques un polymère filmogène comprenant des fonctions réactives choisies parmi les fonctions hydroxyle, amine primaire et secondaire, thiol, acide carboxylique et anhydride carboxylique, et
• une deuxième étape consistant à appliquer sur les fibres kératiniques entourées dudit polymère filmogène, une suspension de nanoparticules métalliques organomodifiées, portant à leur surface une monocouche autoassemblée de groupes organosoufrés de formule
-S-R-R¹
dans laquelle
R a la signification indiquée dans la revendication 4, et
R¹ représente une fonction organique capable de réagir avec les fonctions réactives dudit polymère filmogène,
dans des conditions permettant la fixation covalente des nanoparticules par réaction entre la fonction R¹ et les fonctions réactives dudit polymère filmogène.

6. Procédé selon les revendications 4 ou 5, **caractérisé par le fait que** R¹ représente une fonction époxyde, aziridine, vinyle, acrylonitrile, acide (méth)acrylique, (méth)acrylate d'alkyle, acide crotonique, acide cinnamique et cinnamate d'alkyle, styrène, butadiène, vinyloxy, vinylcétone, maléate d'alkyle, maléimides, vinylsulfone, acide carboxylique, chlorure d'acide carboxylique, anhydride carboxylique, carboxylate d'alkyle, acétal, hémi-acétal, aminal, hémi-aminal, cétone, α-hydroxycétone, α-halogénocétone, lactone, thiolactone, isocyanate, thiocyanate, imine, imide (succinimides, glutimides), ester de N-hydroxysuccinimide, imidate, oxazine, oxazoline, oxazinium, oxazilinium, halogénures d'alkyle, d'aryle ou d'aralkyle, halogénure d'un carbocycle ou hétérocycle insaturé (par exemple chlorotriazine, chlorpyrimidine, chloroquinoxaline, chlorobenzotriazole), halogénure de sulfonyle, siloxane, silane, hydrazine, phénylglyoxal, aldéhyde, azlactone, imidoester, thiosulfate, diazirine, pyridylthio, amine primaire et secondaire et phénylazide.

7. Procédé selon la revendication 5, **caractérisé par le fait que** ledit polymère filmogène comportant des fonctions réactives est choisi parmi la polyéthylèneimine, la polylysine, les alcools polyvinyliques, le poly((méth)acrylate d'hydroxyéthyle), les hydroxyalkylcelluloses, l'acide polyacrylique, les polyvinylimidazoles, les polypropylène-imines, les polyallylamines, le chitosane, les carboxyalkylcelluloses, les aminoalkylcelluloses, les polymères dérivés d'acide ou d'anhydride maléique, fumarique et/ou itaconique, les polyamidoamines.

8. Procédé selon la revendication 3, **caractérisé par le fait que** les groupes organosoufrés correspondent à la formule
-S-R-R²
dans laquelle
R a la signification indiquée dans la revendication 4, et
R² représente une fonction organique capable d'établir des interactions faibles avec les fibres kératiniques,
et que l'on applique la suspension de nanoparticules organomodifiées directement sur les fibres kératiniques dans des conditions permettant l'adsorption des nanoparticules par interaction faible entre la fonction R² et la surface du cheveux.

9. Procédé selon la revendication 3, **caractérisé par le fait qu'**il comprend
• une première étape consistant à déposer sur les fibres kératiniques un polymère filmogène et
• une deuxième étape consistant à appliquer sur les fibres kératiniques entourées dudit polymère filmogène une suspension de nanoparticules métalliques organomodifiées portant à leur surface une monocouche autoassemblée de groupes organosoufrés de formule
-S-R-R²
dans laquelle
R a la signification indiquée dans la revendication 4, et
R² représente une fonction organique capable d'établir des interactions faibles avec ledit polymère filmogène,
dans des conditions permettant l'établissement d'interactions faibles entre le groupe R² et ledit polymère filmogène.

10. Procédé selon la revendication 8 et 9, **caractérisé par le fait que** R² représente un groupe dérivé d'un composé choisi parmi les acides carboxyliques et leurs sels, les amines primaires, secondaires, tertiaires ou quaternaires, les phosphates, les composés soufrés oxydés, les fullerènes, les nanotubes de carbone, les composés aromatiques carbocycliques ou hétérocycliques, les pyrènes, les stilbènes, les ferrocènes, les carbazoles, l'uréido-pyrimidone, la mélamine, l'acide cyanurique, les phtalohydrazides, l'isoguanine, le glycolurile, l'uracile, l'acylaminopyridine, la thymine, la guanine, la cytidine, l'adénine et la ptérine.

11. Procédé selon la revendication 9, **caractérisé par le fait que** le polymère filmogène est choisi parmi la polyéthylèneimine, la polylysine, les alcools polyvinyliques, le poly((méth)acrylate d'hydroxyéthyle), les hydroxyalkylcelluloses, l'acide polyacrylique, les polyvinylimidazoles, les polypropylène-imines, les polyallylamines, le chitosane, les carboxyalkylcelluloses, les aminoalkylcelluloses, les polymères dérivés d'acide ou d'anhydride maléique, fumarique et/ou itaconique, les polyamidoamines.

12. Procédé selon la revendication 3, **caractérisé par le fait que** la suspension de nanoparticules métalliques organomodifiées contient en outre au moins un polymère filmogène à l'état dissous ou dispersé dans le milieu cosmétiquement acceptable.

13. Procédé selon la revendication 10, **caractérisé par le fait que** le polymère filmogène est choisi parmi la polyéthylèneimine, la polylysine, les alcools polyvinyliques, le poly((méth)acrylate d'hydroxyéthyle), les hydroxyalkylcelluloses, l'acide polyacrylique, les polyvinylimidazoles, les polypropylène-imines, les polyallylamines, le chitosane, les carboxyalkylcelluloses, les aminoalkylcelluloses, les polymères dérivés d'acide ou d'anhydride maléique, fumarique et/ou itaconique, les polyamidoamines.

14. Procédé selon la revendication 3, **caractérisé par le fait que** les composés organosoufrés portent des fonctions susceptibles de réagir entre elles de manière à former autour de la fibre, lors de l'évaporation du milieu solvant, une couche de matériau réticulé.

15. Procédé selon la revendication 14, **caractérisé par le fait que** les fonctions susceptibles de réagir entre elles sont choisies parmi les alcoxysilanes, les halogénosilanes, les siloxanes, les thiols et les doubles liaisons acryliques ou vinyliques.

16. Procédé selon la revendication 3, **caractérisé par le fait que** les nanoparticules métalliques organomodifiées sont enrobées d'un polymère organique.

17. Procédé selon la revendication 16, **caractérisé par le fait que** le polymère organique est adsorbé à leur surface.

18. Procédé selon la revendication 16, **caractérisé par le fait que** le polymère organique est fixé par liaison covalente aux composés organosoufrés autoassemblés.

19. Utilisation et procédé selon l'une quelconque des revendications précédentes, **caractérisés par le fait que** les métaux formant les nanoparticules métalliques organomodifiées sont choisis parmi les métaux alcalins, les métaux alcalino-terreux, les métaux de transition, les métaux des terres rares, et des alliages de ces métaux.

20. Utilisation et procédé selon la revendication 19, **caractérisés par le fait que** les métaux formant les nanoparticules métalliques organomodifiées sont choisis parmi l'aluminium, le cuivre, le cadmium, le sélénium, l'argent, l'or, l'indium, le fer, le platine, le nickel, le molybdène, le silicium, le titane, le tungstène, l'antimoine, le palladium, le zinc, l'étain et les alliages de ces métaux.

21. Utilisation et procédé selon la revendication 20, **caractérisés par le fait que** les métaux formant les nanoparticules métalliques organomodifiées sont choisis parmi l'or, l'argent, le palladium, le platine, le cadmium, le sélénium et les alliages de ces métaux.

22. Utilisation et procédé selon l'une quelconque des revendications précédentes, **caractérisés par le fait que** les nanoparticules métalliques organomodifiées ont une structure de type noyau/enveloppe avec une enveloppe métallique entourant un noyau dont le matériau est différent d'un métal à l'état élémentaire.

23. Utilisation et procédé selon la revendication 22 **caractérisé par le fait que** le matériau formant le noyau est un matériau minéral choisi parmi les oxydes, oxydes dihydratés, hydroxydes, carbonates, sulfures, silicates et phosphates de silicium, calcium, magnésium, zinc, aluminium, titane, zirconium ou cérium, les micas et les nacres.

24. Procédé selon la revendication 22, **caractérisé par le fait que** matériau formant le noyau est un polymère organique choisi parmi les homopolymères et copolymères de styrène, les polyorganosiloxanes, les polymères fluorés, les copolymères d'éthylène et d'acétate de vinyle, l'alcool polyvinylique, le poly(oxyde d'éthylène), la polyvinylpyrrolidone, les homopolymères et copolymères à base d'acide (méth)acrylique et de (méth)acrylates d'alkyle, les polyuréthannes, les polyamides, les polycarbonates, le poly(chlorure de vinyle), le poly(acétate de vinyle), le polypropylène, le polyéthylène, le polyisobutylène, le poly(1-buténylène), les éthers de cellulose, les esters organiques de cellulose, les carboxyalkylcelluloses, les sulfates de cellulose, les sulfates de dextrane et les éthers de dextranes.

25. Utilisation et procédé selon l'une quelconque des revendications précédentes, **caractérisés par le fait que** les nanoparticules ont une forme sphérique, lamellaire, fibrillaire ou aléatoire.

26. Utilisation et procédé selon l'une quelconque des revendications précédentes, **caractérisés par le fait que** les nanoparticules métalliques ont une taille moyenne comprise entre 1 nm et 500 nm, de préférence entre 1 nm et 100 nm et plus particulièrement entre 1 nm et 50 nm.

27. Utilisation et procédé selon l'une quelconque des revendications précédentes, **caractérisés par le fait que** les nanoparticules métalliques sont modifiées par greffage de groupes absorbant la lumière visible ou UV, dérivés de colorants organiques choisis parmi les colorants benzéniques nitrés, les colorants benzéniques aminés, les colorants azoïques, les colorants naphto-, benzo- ou anthraquinoniques, les colorants de type diamine aromatique, les aminophénols, les colorants phénoliques et naphtoliques, les porphyrines telles que les tétraphénylporphyrines et les métalloporphyrines, les phtalocyanines, les caroténoïdes, les flavonoïdes et différentes molécules fluorescences telles que la fluorescéine, la rhodamine et la coumarine.

28. Procédé de pré-traitement ou de post-traitement des fibres kératiniques humaines, consistant à mettre en oeuvre le procédé de coloration et/ou de traitement selon l'une quelconque des revendications 3 à 27, respectivement avant ou après un traitement, de coloration d'oxydation, de réduction, de décoloration, de permanente, de coiffage ou de défrisage.

29. Composition cosmétique contenant, dans un milieu cosmétiquement acceptable,
• au moins un principe actif cosmétique, et
• des nanoparticules métalliques organomodifiées portant à leur surface une monocouche autoassemblée de composés organosoufrés, décrites dans les revendications 3 à 27.

30. Composition cosmétique selon la revendication 29, **caractérisée par le fait qu'**elle contient de 0,0001 % à 50 % en poids, en particulier de 0,01 % et 5 % en poids, et idéalement de 0,05 % à 2 % en poids, de nanoparticules métalliques organomodifiées.

31. Composition cosmétique selon l'une des revendications 29 et 30, **caractérisée par le fait que** les principes actifs cosmétiques sont choisis parmi les vitamines, les saccharides, les oligosaccharides, les polysaccharides hydrolysés ou non, modifiés ou non, les acides aminés, les oligopeptides, les peptides, les protéines hydrolysées ou non, modifiées ou non, les polyacides aminés, les enzymes, les acides et alcools gras ramifiés ou non, les cires animales, végétales ou minérales, les céramides et les pseudo-céramides, les acides organiques hydroxylés, les filtres UV, les agents anti-oxydants et les agents anti-radicaux libres, les agents chélatants, les agents antipelliculaires, les agents régulateurs de séborrhée, les agents apaisants, les agents tensioactifs cationiques, les polymères cationiques, les polymères amphotères, les silicones organomodifiées ou non, les huiles minérales, végétales ou animales, les polyisobutènes et poly(α-oléfines), les esters gras, les polymères anioniques sous forme dissoute ou dispersée, les polymères non-ioniques sous forme dissoute ou dispersés, les agents réducteurs, les colorants capillaires ou les pigments et leurs mélanges.

32. Composition cosmétique selon l'une quelconque des revendications 29 à 31, **caractérisée par le fait que** le principe actif cosmétique est présent à raison de 0,001 à 50 % en poids, de préférence à raison de 0,01 à 20 % en poids, et en particulier à raison de 0,1 à 10 % en poids, rapporté au poids total de la composition cosmétique.
